# EUROPEAN PATENT APPLICATION

(11) **EP 4 748 358 A1**
(43) Date of publication of application: **27.05.2026**
(21) Application number: 24849223.3
(22) Date of filing: 31.07.2024
(51) Int. Cl.: A61F 13/51, A61F 13/15, A61F 13/49, A61F 13/496

(54) **MANUFACTURING METHOD FOR WEARING ARTICLE AND MANUFACTURING DEVICE FOR WEARING ARTICLE**

(30) Priority: 01.08.2023 JP 2023125833
(71) Applicant: Zuiko Corporation, Ibaraki-shi Osaka 5670082 (JP)
(72) Inventor: UMEBAYASHI, Toyoshi, Ibaraki-shi, Osaka 567-0082 (JP)
(74) Representative: SSM Sandmair
(86) International application number: PCT/JP2024/027407
(87) International publication number: WO 2025/028578

(57) **Abstract**

Providing a manufacturing method and a manufacturing apparatus for a wearing article with which a wearing article in which a pocket is formed can be manufactured relatively simply. The manufacturing method for a wearing article includes a laminate forming step, an absorbent body arranging step, an adhesive applying step, and a bonding step. In the laminate forming step, an elastic member is arranged on a first area, having a strip-like shape, on a first sheet, and then a laminate in which the elastic member is covered by a second sheet is formed. In the absorbent body arranging step, an absorbent body is arranged on a first surface of the laminate. In the adhesive applying step, a second area on which an adhesive is applied is formed on the first surface of the laminate. In the bonding step, the first area and the second area are folded along a longitudinal direction such that the first area covers an end edge of the absorbent body, and then the second area is bonded to the first surface. The second area includes: a first portion formed with a strip-like shaped gap that is between the first portion and a first end edge, and that overlaps the absorbent body in the bonding step; and a second portion formed to be adjacent to the gap in the longitudinal direction and be in contact with the first end edge.

## Description

### Technical Field

The present disclosure relates to a manufacturing apparatus for a wearing article and a manufacturing method for a wearing article.

### Background Art

Patent Literature 1 (JP H04-354948 A) discloses a wearing article such as a disposable diaper including a three-dimensional cuff (pocket) that prevents leakage of bodily waste from a waist opening, and a manufacturing method therefor.

The wearing article disclosed in Patent Literature 1 includes a backsheet, a topsheet, and an absorbent body arranged between the backsheet and the topsheet. The three-dimensional cuff of the wearing article is provided on an inner side of the waist opening to extend toward the absorbent body.

The manufacturing method for a wearing article disclosed in Patent Literature 1 includes a step of intermittently arranging absorbent bodies on a web-shaped backsheet fed from an unwinding device (absorbent body arranging step), a step of overlapping a topsheet fed from a roll on the backsheet and the absorbent bodies (overlapping step), a step of arranging cuff elastic members and wrapping the cuff elastic members with both end edge portions of the backsheet (cuff forming step), and a step of arranging waist elastic members and wrapping the waist elastic members by using again both end edge portions of the backsheet (waist elastic member arranging step).

### Citation List

### Patent Literature

Patent Literature 1: JP H04-354948 A

### Summary of Invention

### Technical Problems

In the manufacturing method disclosed in Patent Literature 1, the step of wrapping the elastic members with the sheet (each of the cuff forming step and the waist elastic member arranging step) is repeated in order to form the pocket, and thus the steps and the manufacturing apparatus are likely to be complicated.

It is an object of the present disclosure to provide a manufacturing method and a manufacturing apparatus for a wearing article with which a wearing article in which a pocket is formed can be manufactured relatively simply.

### Solutions to Problems

A wearing article manufactured by a manufacturing method for a wearing article of the present disclosure includes: a waist member having a strip-like shape, the waist member having a pocket formed therein, the waist member being configured to be positioned around a waist of a wearer; and an absorbent body having a strip-like shape, the absorbent body being joined to the waist member, the absorbent body being configured to be positioned at a crotch of the wearer.

The manufacturing method for a wearing article comprises a laminate forming step, an absorbent body arranging step, an adhesive applying step, and a bonding step.

In the laminate forming step, an elastic member extending along the longitudinal direction is arranged on a first area on a first sheet conveyed in a longitudinal direction, the first area having a strip-like shape and including an end edge extending in the longitudinal direction which overlaps a first end edge in a width direction of the first sheet, then, a laminate in which the elastic member arranged on the first sheet is covered by a second sheet is formed. In the absorbent body arranging step, the absorbent body is arranged on a first surface of the laminate to overlap a part of a second end edge that is another end with respect to the first end edge. In the adhesive applying step, a second area on which an adhesive is applied in a strip-like shape along the longitudinal direction is formed on the first surface of the laminate. In the bonding step, the first area and the second area are folded along the longitudinal direction such that the first area covers an end edge of the absorbent body joined to the laminate, and then the second area is bonded to the first surface.

The second area includes a first portion and a second portion. The first portion is an area formed with a gap between the first portion and the first end edge in plan view, the gap having a strip-like shape extending along the longitudinal direction and being configured to overlap the absorbent body in the bonding step. The second portion is an area formed to be adjacent to the gap in the longitudinal direction and be in contact with the first end edge in plan view.

A manufacturing apparatus for a wearing article of the present disclosure comprises a laminate forming part, an absorbent body arranging part, an adhesive applying part, and a bonding part.

The laminate forming part is configured to arrange, on a first area on a first sheet conveyed in a longitudinal direction, an elastic member extending along the longitudinal direction, the first area having a strip-like shape and including an end edge extending in the longitudinal direction which overlaps a first end edge in a width direction of the first sheet, and is configured to then form a laminate in which the elastic member arranged on the first sheet is covered by a second sheet. The absorbent body arranging part is configured to arrange, on a first surface of the laminate, the absorbent body to overlap a part of a second end edge that is another end with respect to the first end edge. The adhesive applying part is configured to form, on the first surface of the laminate, a second area on which an adhesive is applied in a strip-like shape along the longitudinal direction. The bonding part is configured to fold the first area and the second area along the longitudinal direction such that the first area covers an end edge of the absorbent body joined to the laminate, and then bond the second area to the first surface.

The second area includes a first portion and a second portion. The first portion is an area formed with a gap between the first portion and the first end edge in plan view, the gap having a strip-like shape extending along the longitudinal direction and being configured to be overlapped with the absorbent body by the bonding part. The second portion is an area formed to be adjacent to the gap in the longitudinal direction and be in contact with the first end edge in plan view.

### Advantageous Effects of Invention

According to a manufacturing method and a manufacturing apparatus according to the present disclosure, a wearing article in which a pocket is formed can be manufactured relatively simply.

### Brief Description of Drawings

[Fig. 1] It is a perspective view of a disposable diaper 1.
[Fig. 2] It is a developed view of the disposable diaper 1 in a state where two waist members 2 are not joined, the view viewing a surface that comes into contact with the skin of a wearer.
[Fig. 3] It is a plan view of a laminate 2a.
[Fig. 4A] It is a schematic process view illustrating a manufacturing apparatus 100 and a manufacturing method for the disposable diaper 1.
[Fig. 4B] It is a schematic process view illustrating the manufacturing apparatus 100 and the manufacturing method for the disposable diaper 1.
[Fig. 5] It is a plan view illustrating elastic members 23 in the laminate 2a that has passed through an elastic member fixing step.
[Fig. 6] It is a plan view illustrating the laminate 2a and an absorbent body 3 that have passed through an adhesive applying process.

### Description of Embodiment

A manufacturing method and a manufacturing apparatus 100 for a disposable diaper 1 according to an embodiment of the present disclosure will be described.

### (1) Disposable Diaper

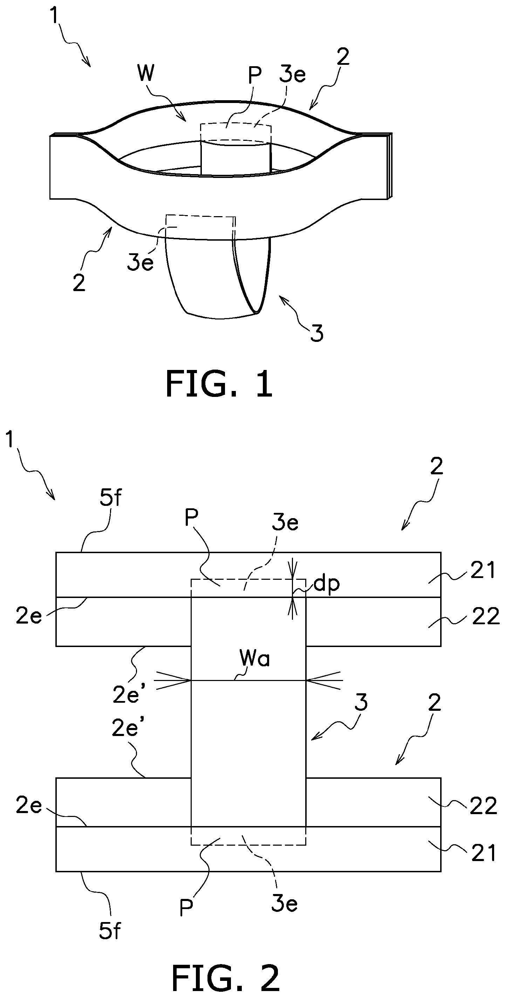

First, the disposable diaper 1 will be described. The disposable diaper 1 is an example of a wearing article. Fig. 1 is a perspective view of the disposable diaper 1. The disposable diaper 1 includes two waist members 2 and an absorbent body 3. A pocket P is formed in the waist member 2. As illustrated in Fig. 1, end portions of the two waist members 2 in a longitudinal direction are joined to each other, and the two waist members 2 form a waist opening W into which the torso of a wearer is fit. Two end portions 3e of the absorbent body 3 are each joined to a corresponding one of the respective middles of the two waist members 2 in the longitudinal direction thereof. Fig. 2 is a developed view of the disposable diaper 1 in a state where the two waist members 2 are not joined, the view viewing a surface that comes into contact with the skin of the wearer.

### (1-1) Waist Member

The waist member 2 is a laminate that has a strip-like shape, that has the pocket P formed therein, and that is positioned around the waist of the wearer when the wearer wears the disposable diaper 1. The waist member 2 includes a first sheet 21, a second sheet 22, a first elastic member 23a, a second elastic member 23b, and a third elastic member 23c.

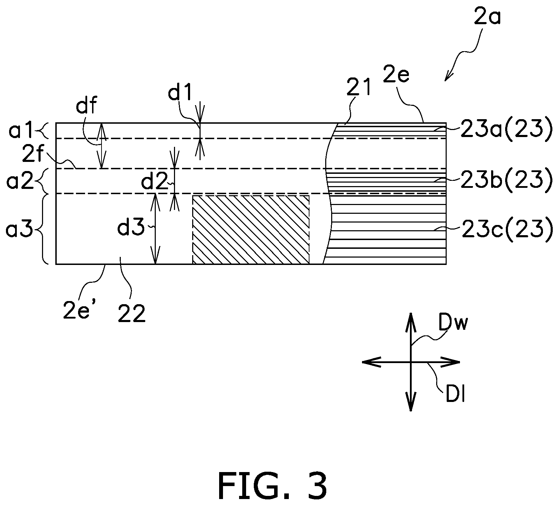

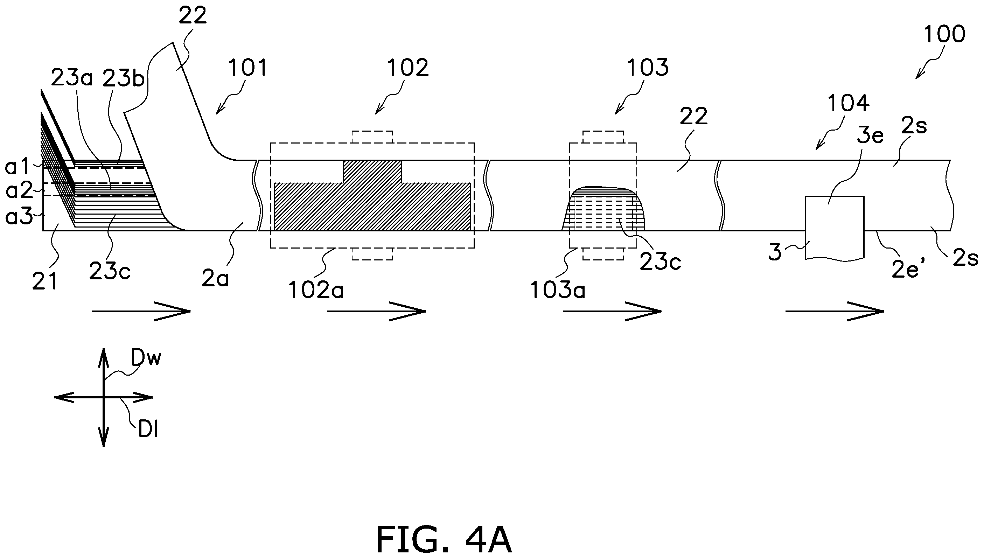

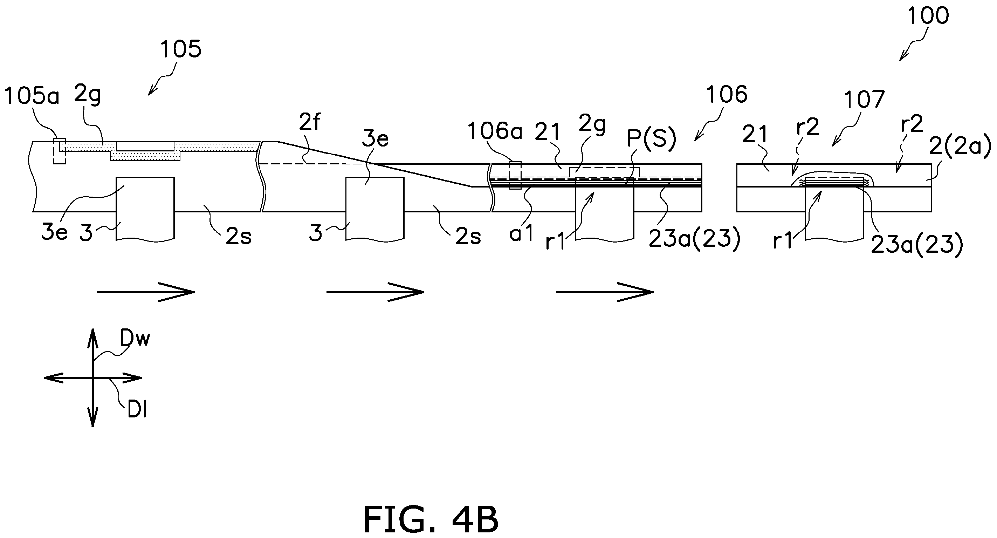

Although details will be described later, the waist member 2 is formed by folding a laminate 2a in which the first elastic member 23a, the second elastic member 23b, and the third elastic member 23c are sandwiched between the first sheet 21 and the second sheet 22, at a predetermined position. Fig. 3 is a plan view of the laminate 2a. In the following description, a longitudinal direction Dl and a width direction Dw of each of the laminate 2a, the first sheet 21, the second sheet 22, or the waist member 2 correspond to directions indicated by arrows in Figs. 3, 4A, 4B, 5, and 6. Note that in Fig. 2, for convenience, a part of the second sheet 22 is illustrated in a transparent manner. In addition, in Fig. 2, an area in which the absorbent body 3 is arranged is indicated by hatching including oblique lines.

Hereinafter, one end edge in the width direction Dw of the laminate 2a is referred to as a first end edge 2e, and the other end edge is referred to as a second end edge 2e'. In addition, a position where the laminate 2a is folded when the waist member 2 is formed is referred to as a folding line 2f. The folding line 2f is a line extending in the longitudinal direction Dl. Although a distance df in the width direction Dw of the laminate 2a between the folding line 2f and the first end edge 2e is not limited to the following, the distance df is about 1/3 of the length in the width direction Dw of the laminate 2a.

The pocket P prevents bodily waste of the wearer from leaking from the waist opening W. The pocket P is formed to open downward when the wearer wears the disposable diaper 1. Although details will be described later, a part overlapping in plan view the absorbent body 3, in the laminate 2a folded along the folding line 2f is the pocket P.

### (1-1-1) First Sheet and Second Sheet

The first sheet 21 and the second sheet 22 are rectangular sheets formed in the same size and the same shape. The first sheet 21 and the second sheet 22 are each formed using non-woven fabric. The first sheet 21 and the second sheet 22 are overlaid such that their end edges overlap each other.

### (1-1-2) First Elastic Member, Second Elastic Member, and Third Elastic Member

The first elastic member 23a, the second elastic member 23b, and the third elastic member 23c are thread-like elastic members arranged to be stretchable along the longitudinal direction Dl of the laminate 2a. Hereinafter, the first elastic member 23a, the second elastic member 23b, and the third elastic member 23c are also collectively referred to as elastic members 23. The elastic member 23 is formed using a material such as polyurethane, natural rubber, or thermoplastic resin. In the present embodiment, the elastic member 23 is a rubber thread.

One or more first elastic members 23a are arranged on a first arrangement area a1. The first arrangement area a1 is an area, on the first sheet 21, having a strip-like shape and extending in the longitudinal direction Dl of the first sheet 21 (that is, the longitudinal direction Dl of the laminate 2a). A width d1 of the first arrangement area a1 in the width direction Dw of the laminate 2a is set to be equal to or smaller than a depth dp of the pocket P in the width direction Dw of the waist member 2. One of end edges, of the first arrangement area a1, extending in the longitudinal direction Dl of the first sheet 21 overlaps the first end edge 2e. The first arrangement area a1 is an example of a first area.

One or more second elastic members 23b are arranged on a second arrangement area a2. The second arrangement area a2 is an area having a strip-like shape and extending in the longitudinal direction Dl of the laminate 2a, on the first sheet 21. A width d2 of the second arrangement area a2 in the width direction Dw of the laminate 2a is set to be equal to or smaller than a difference between the distance df and the depth dp. An end edge, of the second arrangement area a2, extending in the longitudinal direction Dl of the first sheet 21 and located on the first end edge 2e side overlaps the folding line 2f.

One or more third elastic members 23c are arranged on a third arrangement area a3. The third arrangement area a3 is an area having a strip-like shape and extending in the longitudinal direction Dl of the laminate 2a, on the first sheet 21. A width d3 of the third arrangement area a3 in the width direction Dw of the laminate 2a is set to be equal to or smaller than a distance in the width direction Dw of the first sheet 21 between the second end edge 2e' and an end edge of the absorbent body 3 joined to the laminate 2a. One of end edges, of the third arrangement area a3, extending in the longitudinal direction Dl of the first sheet 21 overlaps the second end edge 2e'.

### (1-2) Absorbent Body

The absorbent body 3 is a laminate that has a strip-like shape, that is joined to the waist member 2, and that is positioned at the crotch of the wearer when the wearer wears the disposable diaper 1. The absorbent body 3 absorbs bodily fluid such as urine of the wearer. The absorbent body 3 is formed, for example, by sandwiching an absorbent core (not illustrated) that absorbs bodily fluid, between two sheets having the same size and the same shape (strip-like shape), and by joining end edges of the two sheets to each other.

As illustrated in Fig. 2, the end portion 3e of the absorbent body 3 in the longitudinal direction of the absorbent body 3 is arranged at the middle of the waist member 2 in the longitudinal direction Dl, and the absorbent body 3 is joined.

### (2) Manufacturing Apparatus and Manufacturing Method for Disposable Diaper

Next, the manufacturing apparatus 100 and the manufacturing method for the disposable diaper 1 will be described. The manufacturing apparatus 100 for the disposable diaper 1 includes a laminate forming part 101, an elastic member fixing part 102, an elastic member cutting part 103, an absorbent body arranging part 104, an adhesive applying part 105, a bonding part 106, and a laminate cutting part 107. Figs. 3A and 3B are schematic process views each illustrating the manufacturing apparatus 100 and the manufacturing method for the disposable diaper 1.

Note that one of the two waist members 2 and one of the two laminates 2a will be described below for convenience, but a similar manufacturing method is used for the other one of the waist members 2 and the other one of the laminates 2a, by using a similar manufacturing apparatus 100.

### (2-1) Laminate Forming Part

The laminate forming part 101 performs a laminate forming step. In the laminate forming step, first, the first elastic member 23a extending along the longitudinal direction Dl is arranged on the first arrangement area a1 on the first sheet 21 conveyed in the longitudinal direction Dl, the second elastic member 23b extending along the longitudinal direction Dl is arranged on the second arrangement area a2, and the third elastic member 23c extending along the longitudinal direction Dl is arranged on the third arrangement area a3. Next, the laminate 2a in which the elastic members 23 arranged on the first sheet 21 are covered by the second sheet 22 is formed.

When the laminate forming step is completed, the laminate 2a is conveyed to the elastic member fixing part 102.

### (2-2) Elastic Member Fixing Part

The elastic member fixing part 102 performs an elastic member fixing step. In the elastic member fixing step, at least a part of the elastic member 23 is fixed to at least one of the first sheet 21 and the second sheet 22.

More specifically, in the elastic member fixing step, a fixed range r1 and a non-fixed range r2 are formed for the first elastic member 23a. The fixed range r1 is an example of a first range. The non-fixed range r2 is an example of a second range.

The fixed range r1 is a range in which the first elastic member 23a is fixed to at least one of the first sheet 21 and the second sheet 22 along the longitudinal direction Dl of the laminate 2a. A width of the fixed range r1 in the longitudinal direction of the laminate 2a is the same as a width Wa (see Fig. 2) of the absorbent body 3 in the longitudinal direction of the laminate 2a. In addition, the fixed range r1 is formed to correspond to a position where the absorbent body 3 is arranged in the absorbent body arranging step to be described later, in the longitudinal direction of the laminate 2a. As a result, the fixed range r1 overlaps the absorbent body 3 in a bonding step to be described later.

The non-fixed range r2 is a range that is adjacent to the fixed range r1 in the longitudinal direction Dl of the laminate 2a, and in which the first elastic member 23a is not fixed to either the first sheet 21 or the second sheet 22 along the longitudinal direction Dl of the laminate 2a. In other words, the non-fixed range r2 is a portion other than the fixed range r1 for the first elastic member 23a.

The second elastic member 23b and the third elastic member 23c are fixed to at least one of the first sheet 21 and the second sheet 22 without interruption along the longitudinal direction Dl of the laminate 2a.

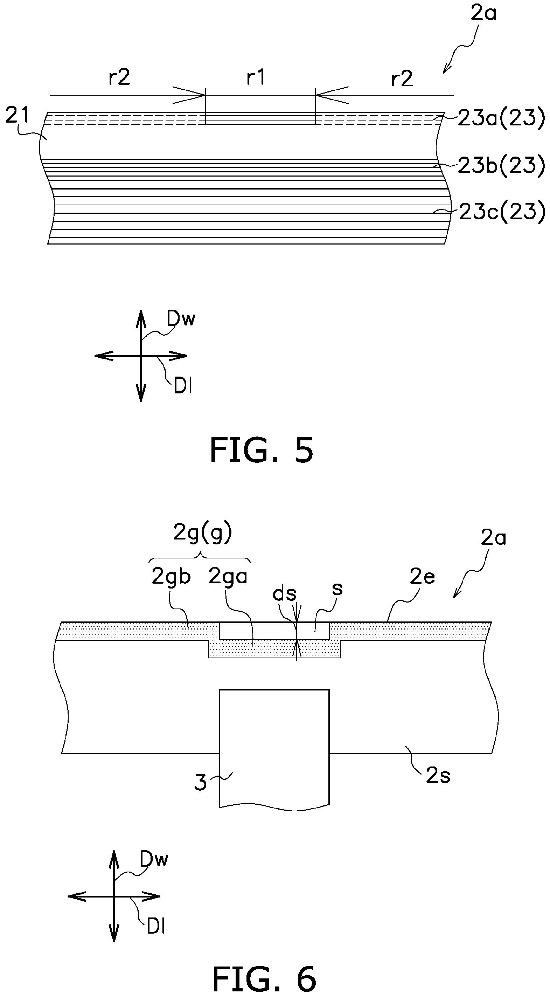

Fig. 5 is a plan view illustrating the elastic members 23 in the laminate 2a that has passed through the elastic member fixing . In Fig. 5, for convenience, the second sheet 22 is illustrated in a transparent manner. In addition, in Fig. 5, the elastic members 23 fixed to at least one of the first sheet 21 and the second sheet 22 are indicated by solid lines, and the elastic members 23 not fixed to either the first sheet 21 or the second sheet 22 are indicated by dashed lines.

When the elastic member fixing step is completed, the laminate 2a is conveyed to the elastic member cutting part 103.

In the present embodiment, the elastic member fixing part 102 includes an ultrasonic horn (not illustrated) and an anvil roller 102a. The elastic member fixing part 102 can perform ultrasonic welding between the elastic member 23 and at least one of the first sheet 21 and the second sheet 22 by using the ultrasonic horn and the anvil roller 102a. In Fig. 3a, a range in which ultrasonic vibration is applied to the laminate 2a is indicated by hatching including oblique lines.

The ultrasonic horn includes a vibration surface on which ultrasonic vibration occurs. The ultrasonic horn applies ultrasonic vibration to the laminate 2a that comes into contact with the vibration surface. The anvil roller 102a is disposed to sandwich the laminate 2a together with the vibration surface of the ultrasonic horn. The anvil roller 102a has a cylindrical shape, and rotates about the central axis in accordance with the conveyance speed of the laminate 2a.

### (2-3) Elastic Member Cutting Part

The elastic member cutting part 103 performs an elastic member cutting step. In the elastic member cutting step, an elastic force of the third elastic member 23c to be overlapped in plan view with the absorbent body 3 to be arranged in the absorbent body arranging part 104 is disabled (brought into a state having almost no elastic force). Specifically, the third elastic member 23c to be overlapped in plan view with the absorbent body 3 to be arranged in the absorbent body arranging part 104 is cut at a plurality of positions with predetermined gaps along the longitudinal direction Dl of the laminate 2a. In Fig. 3a, for convenience, the third elastic member 23c is illustrated by illustrating a part of the second sheet 22 in a transparent manner.

When the elastic member cutting step is completed, the laminate 2a is conveyed to the absorbent body arranging part 104.

The elastic member cutting part 103 includes two rolls 103a that sandwich the conveyed laminate 2a in a lamination direction. The roll 103a cuts a predetermined portion of the third elastic member 23c while rotating in accordance with the conveyance speed of the laminate 2a.

### (2-4) Absorbent Body Arranging Part

The absorbent body arranging part 104 performs an absorbent body arranging step. In the absorbent body arranging step, the end portion 3e of the absorbent body 3 is arranged on a first surface 2s, which is one of surfaces of the laminate 2a, to overlap a part of the second end edge 2e', and is joined.

When the absorbent body arranging step is completed, the laminate 2a is conveyed to the adhesive applying part 105.

### (2-5) Adhesive Applying Part

The adhesive applying part 105 performs an adhesive applying step. In the adhesive applying step, a bonding area 2g on which an adhesive g is applied in a strip-like shape along the longitudinal direction Dl of the laminate 2a is formed on the first surface 2s of the laminate 2a. The bonding area 2g includes a first bonding area 2ga and a second bonding area 2gb. Fig. 6 is a plan view illustrating the laminate 2a and the absorbent body 3 that have passed through the adhesive applying step. The bonding area 2g is an example of a second area.

The first bonding area 2ga is formed by applying the adhesive g in a strip-like shape along the longitudinal direction Dl of the laminate 2a while a gap s having a strip-like shape and extending along the longitudinal direction Dl is formed between the first bonding area 2ga and the first end edge 2e in plan view. The gap s is a part to be formed as the pocket P in the bonding step to be described later. Therefore, the first bonding area 2ga is formed in accordance with the shape of the pocket P. Specifically, the first bonding area 2ga is formed such that a width ds of the gap s is the same as the depth dp of the pocket P, such that the length of the gap s in the longitudinal direction Dl of the laminate 2a is the same as the width Wa of the absorbent body 3 in the longitudinal direction of the laminate 2a, and such that the first bonding area 2ga is positioned at the middle of the waist member 2 in the longitudinal direction Dl. As a result, the gap s can overlap the absorbent body 3 in the bonding step.

The second bonding area 2gb is formed by applying the adhesive g in a strip-like shape along the longitudinal direction Dl of the laminate 2a, to be adjacent to the gap s in the longitudinal direction Dl of the laminate 2a and be in contact with the first end edge 2e in plan view.

As illustrated in Fig. 5, in the present embodiment, the respective widths of the first bonding area 2ga and the second bonding area 2gb are the same, but the respective widths of the first bonding area 2ga and the second bonding area 2gb may be different from each other. The first bonding area 2ga is an example of a first portion. The second bonding area 2gb is an example of a second portion.

When the adhesive applying step is completed, the laminate 2a is conveyed to the bonding part 106.

In the present embodiment, the adhesive g is a hot-melt adhesive. The adhesive applying part 105 includes a spray nozzle 105a that applies the hot-melt adhesive onto the first surface 2s of the laminate 2a to form the bonding area 2g.

### (2-6) Bonding Part

The bonding part 106 performs a bonding step. In the bonding step, first, the first arrangement area a1 and the bonding area 2g are folded along the folding line 2f such that the first arrangement area a1 covers an end edge of the end portion 3e of the absorbent body 3 joined to the laminate 2a. Then, the bonding area 2g is bonded to the first surface 2s of the laminate 2a. The first arrangement area a1 and the bonding area 2g are folded along the folding line 2f, whereby the fixed range r1 and the gap s are brought into a state of overlapping the absorbent body. In Fig. 3b, for convenience, the first elastic member 23a is illustrated by illustrating a part of the first sheet 21 in a transparent manner.

When the bonding step is completed, the laminate 2a is conveyed to the laminate cutting part 107.

In the present embodiment, the bonding part 106 includes a heater 106a that heats the bonding area 2g. When the heater 106a heats the bonding area 2g, the bonding area 2g is bonded to the first surface 2s of the laminate 2a.

The first bonding area 2ga of the bonding area 2g is formed to have the gap s between the first bonding area 2ga and the first end edge 2e in plan view. Therefore, when the first bonding area 2ga is bonded to the first surface 2s, a part of the laminate 2a corresponding to the gap s is not bonded to the first surface 2s, and becomes the pocket P.

### (2-7) Laminate Cutting Part

The laminate cutting part 107 performs a laminate cutting step. In the laminate cutting step, the laminate 2a is cut along the width direction Dw such that the absorbent body 3 joined in the absorbent body arranging part 104 is positioned at the middle of the laminate 2a in the longitudinal direction Dl. The cut laminate 2a becomes the waist member 2.

As described above, the first elastic member 23a is fixed to at least one of the first sheet 21 and the second sheet 22 in the fixed range r1, and is not fixed to either the first sheet 21 or the second sheet 22 in the non-fixed range r2. Therefore, when the laminate 2a is cut in the width direction Dw in the laminate cutting part step, the first elastic member 23a located in the non-fixed range r2 contracts to the fixed range r1. In Fig. 3b, for convenience, the first elastic member 23a is illustrated by illustrating a part of the first sheet 21 in a transparent manner.

Through the steps described above, the disposable diaper 1 in which the two waist members 2 are not joined to each other, illustrated in Fig. 2 is manufactured. Although detailed description is omitted, after the absorbent body 3 is folded such that the two waist members 2 overlap each other, the end portions of the waist members 2 in the longitudinal direction Dl are joined to each other, whereby the disposable diaper 1 illustrated in Fig. 1 is manufactured.

### (3) Characteristics

(3-1)
The disposable diaper 1 includes: the waist member 2, which has a strip-like shape, having the pocket P formed therein and being configured to be positioned around the waist of the wearer; and the absorbent body 3, which has a strip-like shape, being joined to the waist member 2 and being configured to be positioned at the crotch of the wearer. The manufacturing method for the disposable diaper 1 includes the laminate forming step, the absorbent body arranging step, the adhesive applying step, and the bonding step.

In the laminate forming step, the first elastic member 23a extending along the longitudinal direction Dl is arranged on the first arrangement area a1 on the first sheet 21 conveyed in the longitudinal direction Dl, the first arrangement area a1 having a strip-like shape and including the end edge extending in the longitudinal direction Dl which overlaps the first end edge 2e in the width direction Dw of the first sheet 21. In the laminate forming step, then, the laminate 2a in which the first elastic member 23a arranged on the first sheet 21 is covered by the second sheet 22 is formed. In the absorbent body arranging step, the absorbent body 3 is arranged on the first surface 2s of the laminate 2a to overlap a part of the second end edge 2e' that is another end with respect to the first end edge 2e. In the adhesive applying step, a bonding area 2g on which the adhesive g is applied in a strip-like shape along the longitudinal direction Dl is formed on the first surface 2s of the laminate 2a. In the bonding step, the first arrangement area a1 and the bonding area 2g are folded along the longitudinal direction Dl such that the first arrangement area a1 covers the end edge of the absorbent body 3 joined to the laminate 2a, and then the bonding area 2g is bonded to the first surface 2s.

The bonding area 2g includes the first bonding area 2ga and the second bonding area 2gb. The first bonding area 2ga is an area formed with the gap s between the first bonding area 2ga and the first end edge 2e in plan view, the gap s having a strip-like shape extending along the longitudinal direction Dl and being configured to overlap the absorbent body 3 in the bonding step. The second bonding area 2gb is an area formed to be adjacent to the gap s in the longitudinal direction Dl and be in contact with the first end edge 2e in plan view.

In the manufacturing method for the disposable diaper 1 described above, the first elastic member 23a and the second sheet 22 are each continuously arranged on the first sheet 21 at substantially the same position, and thus complication of the steps is reduced. In addition, the first elastic members 23a arranged on the first sheet 21 are held by the second sheet 22 covering them, and thus it is not necessary to fix the first elastic members 23a by folding the laminate 2a, or the like, and complication of the steps is reduced. Moreover, the pocket P is formed by the relatively simple step of folding and bonding a part of the laminate 2a including the bonding area 2g formed with the gap s. Further, since the bonding area 2g includes the second bonding area 2gb, it is not necessary to fold the end edge of the folded laminate 2a in advance, and complication of the steps is reduced.

Therefore, according to the manufacturing method for the disposable diaper 1 described above, the disposable diaper 1 in which the pocket P is provided can be manufactured relatively simply.

(3-2)
The manufacturing method for the disposable diaper 1 further includes an elastic member fixing step of forming the fixed range r1 and the non-fixed range r2.

The fixed range r1 is a range in which the first elastic member 23a is fixed to at least one of the first sheet 21 and the second sheet 22 along the longitudinal direction Dl, and that is configured to overlap the absorbent body 3 in the bonding step. The non-fixed range r2 is a range that is adjacent to the fixed range r1 in the longitudinal direction Dl, and in which the first elastic member 23a is not fixed to either the first sheet 21 or the second sheet 22 along the longitudinal direction Dl.

(3-3)
The manufacturing apparatus 100 for the disposable diaper 1 includes the laminate forming part 101, the absorbent body arranging part 104, the adhesive applying part 105, and the bonding part 106.

The laminate forming part 101 is configured to arrange, on the first arrangement area a1 on the first sheet 21 conveyed in the longitudinal direction Dl, the first elastic member 23a extending along the longitudinal direction Dl, the first arrangement area a1 having a strip-like shape and including the end edge extending in the longitudinal direction Dl which overlaps the first end edge 2e in the width direction Dw of the first sheet 21, and is configured to then form the laminate 2a in which the first elastic member 23a arranged on the first sheet 21 is covered by the second sheet 22.

The absorbent body arranging part 104 is configured to arrange, on the first surface 2s of the laminate 2a, the absorbent body 3 to overlap a part of the second end edge 2e' that is another end with respect to the first end edge 2e. The adhesive applying part 105 is configured to form, on the first surface 2s of the laminate 2a, the bonding area 2g on which the adhesive g is applied in a strip-like shape along the longitudinal direction Dl. The bonding part 106 is configured to fold the first arrangement area a1 and the bonding area 2g along the longitudinal direction Dl such that the first arrangement area a1 covers the end edge of the absorbent body 3 joined to the laminate 2a, and then bond the bonding area 2g to the first surface 2s.

The bonding area 2g includes the first bonding area 2ga and the second bonding area 2gb. The first bonding area 2ga is an area formed with the gap s between the first bonding area 2ga and the first end edge 2e in plan view, the gap s having a strip-like shape extending along the longitudinal direction Dl and being configured to be overlapped with the absorbent body 3 by the bonding part. The second bonding area 2gb is an area formed to be adjacent to the gap s in the longitudinal direction Dl and be in contact with the first end edge 2e in plan view.

In the manufacturing apparatus 100 for the disposable diaper 1 described above, the first elastic member 23a and the second sheet 22 are each continuously arranged on the first sheet 21 at substantially the same position, and thus complication of the apparatus is reduced. In addition, the first elastic members 23a arranged on the first sheet 21 are held by the second sheet 22 covering them, and thus it is not necessary to fix the first elastic members 23a by folding the laminate 2a, or the like, and complication of the apparatus is reduced. Moreover, the pocket P is formed by the relatively simple apparatus with which a part of the laminate 2a including the bonding area 2g formed with the gap s is folded and bonded. Further, since the bonding area 2g includes the second bonding area 2gb, it is not necessary to fold the end edge of the folded laminate 2a in advance, and complication of the apparatus is reduced.

Therefore, according to the manufacturing apparatus for the disposable diaper 1 described above, the disposable diaper 1 in which the pocket P is provided can be manufactured relatively simply.

(3-4)
The manufacturing apparatus 100 for the disposable diaper 1 further includes the elastic member fixing part configured to form the fixed range r1 and the non-fixed range r2.

The fixed range r1 is a range in which the first elastic member 23a is fixed to at least one of the first sheet 21 and the second sheet 22 along the longitudinal direction Dl, and that is configured to be overlapped with the absorbent body 3 by the bonding part. The non-fixed range r2 is a range that is adjacent to the fixed range r1 in the longitudinal direction Dl, and in which the first elastic member 23a is not fixed to either the first sheet 21 or the second sheet 22 along the longitudinal direction Dl.

### (4) Modification

In the embodiment described above, the elastic member 23 is fixed to at least one of the first sheet 21 and the second sheet 22 by ultrasonic welding, but the method of fixing the elastic member 23 is not limited thereto.

For example, welded portions in each of which the first sheet 21 and the second sheet 22 are welded to each other may be provided on both sides in the width direction of the elastic member 23, and the elastic member 23 may be fixed to the first sheet 21 and the second sheet 22 by sandwiching the elastic member 23 in the width direction between the welded portions.

### (5) Summary

Finally, technical ideas that can be grasped from the embodiment described above will be described.

A wearing article manufactured by a manufacturing method for a wearing article, according to a first aspect of the present disclosure includes: a waist member, which has a strip-like shape, having a pocket formed therein and being configured to be positioned around a waist of a wearer; and an absorbent body, which has a strip-like shape, the absorbent body being joined to the waist member and being configured to be positioned at a crotch of the wearer.

The manufacturing method for a wearing article comprises a laminate forming step, an absorbent body arranging step, an adhesive applying step, and a bonding step.

In the laminate forming step, an elastic member extending along the longitudinal direction is arranged on a first area on a first sheet conveyed in a longitudinal direction, the first area having a strip-like shape and including an end edge extending in the longitudinal direction which overlaps a first end edge in a width direction of the first sheet. In the laminate forming step, then, a laminate in which the elastic member arranged on the first sheet is covered by a second sheet is formed. In the absorbent body arranging step, the absorbent body is arranged on a first surface of the laminate to overlap a part of a second end edge that is another end with respect to the first end edge. In the adhesive applying step, a second area on which an adhesive is applied in a strip-like shape along the longitudinal direction is formed on the first surface of the laminate. In the bonding step, the first area and the second area are folded along the longitudinal direction such that the first area covers an end edge of the absorbent body joined to the laminate, and then the second area is bonded to the first surface.

The second area includes a first portion and a second portion. The first portion is an area formed with a gap between the first portion and the first end edge in plan view, the gap having a strip-like shape extending along the longitudinal direction and being configured to overlap the absorbent body in the bonding step. The second portion is an area formed to be adjacent to the gap in the longitudinal direction and be in contact with the first end edge in plan view.

According to this manufacturing method, a disposable diaper in which a pocket is formed can be manufactured with relatively simple steps.

A manufacturing method for a wearing article according to a second aspect of the present disclosure is the manufacturing method for a wearing article according to the first aspect and further comprises an elastic member fixing step. In the elastic member fixing step, a first range and a second range are formed. The first range is a range in which the elastic member is fixed to at least one of the first sheet and the second sheet along the longitudinal direction, and that is configured to overlap the absorbent body in the bonding step. The second range is a range that is adjacent to the first range in the longitudinal direction, and in which the elastic member is not fixed to either the first sheet or the second sheet along the longitudinal direction.

A manufacturing apparatus for a wearing article, according to a third aspect of the present disclosure includes a laminate forming part, an absorbent body arranging part, an adhesive applying part, and a bonding part.

The laminate forming part is configured to arrange, on a first area on a first sheet conveyed in a longitudinal direction, an elastic member extending along the longitudinal direction, the first area having a strip-like shape and including an end edge extending in the longitudinal direction which overlaps a first end edge in a width direction of the first sheet, and is configured to then form a laminate in which the elastic member arranged on the first sheet is covered by a second sheet. The absorbent body arranging part is configured to arrange, on a first surface of the laminate, the absorbent body to overlap a part of a second end edge that is another end with respect to the first end edge. The adhesive applying part is configured to form, on the first surface of the laminate, a second area on which an adhesive is applied in a strip-like shape along the longitudinal direction. The bonding part is configured to fold the first area and the second area along the longitudinal direction such that the first area covers an end edge of the absorbent body joined to the laminate, and then bond the second area to the first surface.

The second area includes a first portion and a second portion. The first portion is an area formed with a gap between the first portion and the first end edge in plan view, the gap having a strip-like shape extending along the longitudinal direction and being configured to be overlapped with the absorbent body by the bonding part. The second portion is an area formed to be adjacent to the gap in the longitudinal direction and be in contact with the first end edge in plan view.

According to this manufacturing apparatus, a disposable diaper 1 in which a pocket is formed can be manufactured relatively simply.

A manufacturing apparatus for a wearing article according to a fourth aspect of the present disclosure is the manufacturing apparatus for a wearing article according to the first aspect and further includes an elastic member fixing part. The elastic member fixing part is configured to form a first range and a second range. The first range is a range in which the elastic member is fixed to at least one of the first sheet and the second sheet along the longitudinal direction, and that is configured to be overlapped with the absorbent body by the bonding part. The second range is a range that is adjacent to the first range in the longitudinal direction, and in which the elastic member is not fixed to either the first sheet or the second sheet along the longitudinal direction.

### Reference Signs List

1: Disposable diaper (wearing article)
2: Waist member
2a: Laminate
2e: First end edge
2e': Second end edge
2f: Folding line
2g: Bonding area (second area)
2ga: First bonding area (first portion)
2gb: Second bonding area (second portion)
2s: First surface
3: Absorbent body
21: First sheet
22: Second sheet
23: Elastic member
100: Manufacturing apparatus
101: Laminate forming part
102: Elastic member fixing part
103: Elastic member cutting part
104: Absorbent body arranging part
105: Adhesive applying part
106: Bonding part
107: Laminate cutting part
a1: First arrangement area (first area)
a2: Second arrangement area
a3: Third arrangement area
Dl: Longitudinal direction
Dw: Width direction
g: Adhesive
P: Pocket
r1: Fixed range (first range)
r2: Non-fixed range (second range)
s: Gap

## Claims

1. A manufacturing method for a wearing article, the wearing article including: a waist member having a strip-like shape, the waist member having a pocket formed therein, the waist member being configured to be positioned around a waist of a wearer; and an absorbent body having a strip-like shape, the absorbent body being joined to the waist member, the absorbent body being configured to be positioned at a crotch of the wearer, the manufacturing method comprising:
a laminate forming step of: arranging, on a first area on a first sheet conveyed in a longitudinal direction, an elastic member extending along the longitudinal direction, the first area having a strip-like shape, the first area including an end edge extending in the longitudinal direction, the end edge of the first area overlapping a first end edge in a width direction of the first sheet; and then forming a laminate in which the elastic member arranged on the first sheet is covered by a second sheet;
an absorbent body arranging step of arranging, on a first surface of the laminate, the absorbent body to overlap a part of a second end edge that is another end with respect to the first end edge;
an adhesive applying step of forming, on the first surface of the laminate, a second area on which an adhesive is applied in a strip-like shape along the longitudinal direction; and
a bonding step of: folding the first area and the second area along the longitudinal direction such that the first area covers an end edge of the absorbent body joined to the laminate; and then bonding the second area to the first surface,
the second area including:
a first portion formed with a gap between the first portion and the first end edge in plan view, the gap having a strip-like shape, the gap extending along the longitudinal direction, the gap being configured to overlap the absorbent body in the bonding step; and
a second portion formed to be adjacent to the gap in the longitudinal direction and be in contact with the first end edge in plan view.

2. The manufacturing method for a wearing article according to claim 1, further comprising an elastic member fixing step of forming:
a first range that is a range in which the elastic member is fixed to at least one of the first sheet and the second sheet along the longitudinal direction, and that is configured to overlap the absorbent body in the bonding step; and
a second range that is a range that is adjacent to the first range in the longitudinal direction, and in which the elastic member is not fixed to either the first sheet or the second sheet along the longitudinal direction.

3. A manufacturing apparatus for a wearing article, the wearing article including: a waist member having a strip-like shape, the waist member having a pocket formed therein, the waist member being configured to be positioned around a waist of a wearer; and an absorbent body having a strip-like shape, the absorbent body being joined to the waist member, the absorbent body being configured to be positioned at a crotch of the wearer, the manufacturing apparatus comprising:
a laminate forming part configured to: arrange, on a first area on a first sheet conveyed in a longitudinal direction, an elastic member extending along the longitudinal direction, the first area having a strip-like shape, the first area including an end edge extending in the longitudinal direction, the end edge of the first area overlapping a first end edge in a width direction of the first sheet; and then form a laminate in which the elastic member arranged on the first sheet is covered by a second sheet;
an absorbent body arranging part configured to arrange, on a first surface of the laminate, the absorbent body to overlap a part of a second end edge that is another end with respect to the first end edge;
an adhesive applying part configured to form, on the first surface of the laminate, a second area on which an adhesive is applied in a strip-like shape along the longitudinal direction; and
a bonding part configured to: fold the first area and the second area along the longitudinal direction such that the first area covers an end edge of the absorbent body joined to the laminate; and then bond the second area to the first surface,
the second area including:
a first portion formed with a gap between the first portion and the first end edge in plan view, the gap having a strip-like shape, the gap extending along the longitudinal direction, the gap being configured to be overlapped with the absorbent body by the bonding part; and
a second portion formed to be adjacent to the gap in the longitudinal direction and be in contact with the first end edge in plan view.

4. The manufacturing method for a wearing article according to claim 1, further comprising an elastic member fixing part configured to form:
a first range that is a range in which the elastic member is fixed to at least one of the first sheet and the second sheet along the longitudinal direction, and that is configured to be overlapped with the absorbent body by the bonding part; and
a second range that is a range that is adjacent to the first range in the longitudinal direction, and in which the elastic member is not fixed to either the first sheet or the second sheet along the longitudinal direction.
